(19) Europäisches Patentamt
European Patent Office
Office européen des brevets

(11) Veröffentlichungsnummer : **0 138 870**
**B1**

# (12) EUROPÄISCHE PATENTSCHRIFT

(45) Veröffentlichungstag der Patentschrift : **12.11.86**

(51) Int. Cl.[4] : **A 61 K 7/50**, C 11 D 1/12

(21) Anmeldenummer : **84901072.3**

(22) Anmeldetag : **10.03.84**

(86) Internationale Anmeldenummer : **PCT/EP 84/00070**

(87) Internationale Veröffentlichungsnummer : **WO/8403622 (27.09.84 Gazette 84/23)**

## (54) STOFFGEMISCH UND WÄSSRIGES HAUTWASCHMITTEL AUS DESSEN BESTANDTEILEN.

(30) Priorität : **16.03.83 DE 3309333**

(43) Veröffentlichungstag der Anmeldung : **02.05.85 Patentblatt 85/18**

(45) Bekanntmachung des Hinweises auf die Patenterteilung : **12.11.86 Patentblatt 86/46**

(84) Benannte Vertragsstaaten : **AT BE CH DE FR GB LI LU NL SE**

(56) Entgegenhaltungen :
**DE-B- 1 213 086**
**GB-A- 1 281 219**
**H. Janistyn: "Handbuch der Kosmetika und Riechstoffe", 3rd edition, vol. 1, "Die kosmetischen Grundstoffe", 1978, Dr. Alfred Hèthig Verlag, Heidelberg (DE) see page 788, Rewomid, Rewopol; page 789, Rewo-Lan 5; page 873, Steinamide; page 874, Steinapol SBFA30, Steinapol SBV; page 875, Steinapol SBF12, Steinapol MPG**
**Seifen, Ölen, Fette, Wachse, Vol. 99, No. 10, May 1973, Augsburg (DE), H. Führer: "Shampoos und Schaumbademittel-Rezeptierung und Parfümierung", page 270ff, see page 271, Schaumbademittel N. 2.4**
**Soap/Cosmetics/Chemical Specialities, Vol. 48, No. 5, May 1972, New York (US), see page 173, examples**

(73) Patentinhaber : **SCHELER, Hermann**
**Rauhbach 1**
**D-6940 Weinheim-Oberflockenbach (DE)**

(72) Erfinder : **LEISING, Erich**
**Kriemhildstr. 26**
**D-6940 Weinheim (DE)**

(74) Vertreter : **Weiss, Ursula, Dr.**
**Gluckstrasse 3**
**D-6800 Mannheim 1 (DE)**

**EP 0 138 870 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Stoffgemisch enthaltend Alkylsulfosuccinat, Fettalkoholpolyglykoläthersuccinat, Fettsäurealkylolamid, Lanolin-Sulfosuccinat, Konservierungsmittel und Wasser. Die Nachteile bekannter Hautwaschmittel, insbesondere für die menschliche Haut, sind vielfältig. Viele bekannte Hautwaschmittel sind nicht gut verträglich, sie reizen insbesondere die Schleimhäute. Weiterhin sind bekannte Hautwaschmittel häufig mit Kochsalz verdickt und mit Parfums und Farbstoffen versetzt. Gerade die letztgenannten Bestandteile reizen eine empfindliche Haut leicht und können außerdem deutliche Allergien verursachen. Daneben besitzt ein parfümiertes Hautwaschmittel noch den Nachteil, daß nach Verwendung desselben mit dem vom Benutzter weiterhin verwendeten Parfum störende Überlagerungsgerüche zu besorgen sind. Auch konzentrieren sich die bekannten Hautwaschmittel im wesentlichen darauf, eine Reinigungswirkung zu erzielen ohne Rücksicht darauf, daß dabei die natürliche Zusammensetzung der Hautoberfläche gleichfalls abgetragen wird. Schon aus den genannten Bestandteilen bekannter Hautwaschmittel läßt sich erkennen, daß sie oft nicht biologisch abbaubar sind. Hinzu kommt noch, daß die herkömmlichen Hautwaschmittel bekannte Seifenbestandteile enthalten, die nicht gut hartwasserverträglich sind, bei denen also Kalkseifen im harten Wasser ausfallen und so die Wirksamkeit des Hautwaschmittels begrenzen. Schließlich können bekannte Hautwaschmittel toxisch sein.

Aus der britischen Patentschrift GB-PS 1 281 219 sind Stoffgemische bekannt, die neben einer Mischung aus Alkylsulfosuccinaten und Acylalkanolamidsulfosuccinaten Fettalkoholpolyglykoläthersulfosuccinate, Fettsäurealkanolamide, Lanolin und Konservierungsmittel enthalten können.

Ausgehend von diesem Stand der Technik liegt der Erfindung die Aufgabe zugrunde, Stoffgemische vorzuschlagen, die durch ihre besondere Zusammensetzung insbesondere als Hautreinigungsmittel geeignet sind, das die Haut schont und nicht allergieträchtig ist. Es soll daher insbesondere für die hautmedizinisch problematische Haut geeignet sein. Daß das Hautwaschmittel dennoch eine gute Reinigungswirkung haben soll, versteht sich von selbst.

Diese Aufgabe wird durch ein Stoffgemisch enthaltend Alkylsulfosuccinat, Fettalkoholpolyglykoläthersuccinat, Fettsäurealkylolamid, Konservierungsmittel und Wasser gelöst, das dadurch gekennzeichnet, ist, daß es aus

a) 450 bis 600 Teilen Dinatriumfettalkoholpolyglykoläthersulfosuccinat der Struktur,

$$R-(-O-CH_2-CH_2-)_n-O-CO-CH_2-\underset{\displaystyle SO_3Na}{\underset{|}{CH}}-COONa$$

mit n insbesondere von 2 bis 12,

b) 30 bis 50 Teilen Dinatriumalkylsulfosuccinat, wobei sich der Alkylrest von einer pflanzlichen Fettsäure ableitet,

c) 30 bis 50 Teile nichtionogenes Fettsäure alkylolamid, insbesondere Cocosfettsäureisopropanolamid der Struktur

$$R-\overset{\displaystyle O}{\overset{\|}{C}}-NH-CH_2-\overset{\displaystyle OH}{\overset{|}{CH}}-CH_3$$

d) 20 bis 40 Teile Lanolin-Sulfosuccinat,

e) 1 Teil Zitronensäure,

f) 1 Teil Konservierungsmittel, frei von Formaldehyd und Formaldehydabspaltern und

g) zu 1 000 Teilen Wasser ergänzt besteht.

Entsprechend den Patentansprüchen 1 und 2 besteht somit das erfindigungsgemäße Hautwaschmittel prozentual gesehen aus 45 bis 60 % des Stoffes gemäß dem obigen Merkmal a), 3 bis 5 % gemäß dem obigen Merkmal b), 3 bis 5 % gemäß dem obigen Merkmal c), 2 bis 4 % gemäß dem obigen Merkmal d), 0,1 % gemäß dem obigen Merkmal e) und 0,1 % gemäß dem obigen Merkmal f), wobei dieses Gemisch durch Wasser zu 100 % als entsprechend wässrige Lösung vervollständigt ist. Dieses Stoffgemisch ist als Hautwaschmittel verwendbar.

Insgesamt läßt sich sagen, daß dieses Stoffgemisch bzw. Hautwaschmittel flüssig ist. Es besitzt einen pH-Wert von ungefähr 6,0. Es ist sehr gut hartwasserverträglich, nicht toxisch und biologisch voll abbaubar. Dieses Stoffgemisch bzw. Hautwaschmittel ist eine klare, stabile Mischung. Bei zu kalter Lagerung kann zwar eine Eintrübung beobachtet werden ; sie ist jedoch bedeutungslos, da sie sich in der Wärme wieder auflöst. Diese Erscheinung ist auch eine Folge der hohen Konzentration, die somit eine volumensparende Konfektionierung des Hautwaschmittels erlaubt.

Es sei darauf hingewiesen, daß erfindungsgemäß kein Parfum und auch kein Farbstoff gegeben ist, der die Haut reizen könnte.

Als bevorzugte Zusammensetzung können folgende Werte genannt werden :

Bestandteil gemäß dem obigen Merkmal a) 54 %, gemäß dem obigen Merkmal b) 4,6 %, gemäß dem obigen Merkmal c) 4,6 %, gemäß dem obigen Merkmal d) 3,6 %, gemäß dem obigen Merkmal e) 0,1 %, gemäß dem obigen Merkmal f) 0,1 % und 33 % Wasser.

In diesem Zusammenhang kann gesagt werden, daß es sich für den Fachmann versteht, daß die Bestandteile gemäß den obigen Merkmalen e) und f), die jeweils zu 0,1 % erfindungsgemäß vorgegeben sein sollen, selbstverständlich nicht unbedingt exakt in dieser Menge beigemischt sein müssen. Dem Fachmann soll durch diese Angabe lediglich ein Anhaltspunkt für die Größenordnung dieser Beimischungen gegeben sein.

Im Ergebnis läßt sich hinsichtlich der Anwendung des erfindungsgemäßen Hautwaschmittels sagen, daß es sich durch eine gute Haut- und Schleimhautverträglichkeit auszeichnet, wie das im Draize-Test beobachtet werden kann. Auch die Augenverträglichkeit ist gut. Die Schaumausbeute in Bezug auf Menge, Stabilität und Qualität ist gleichfalls gut. Gerade wegen dieses guten Schaumbildungsvermögens wird eine verbesserte Waschkraft erzielt. Die abgewaschene Emulsion ist stabilisiert, wozu auch die Hartwasserverträglichkeit beiträgt. Das erfindungsgemäße Hautwaschmittel zieht auf die Haut auf und führt damit zu einem echten, pflegenden Effekt. Dabei wirkt das erfindungsgemäße Hautwaschmittel sogar rückfettend. Dies alles wird noch dadurch unterstützt, daß das Einziehvermögen für danach anzuwendende Hautpflegemittel in den Lipidmantel der Haut beschleunigt wird. Gerade dies ist bei hautmedizinischen Problemfällen von besonderer Bedeutung. Die Alkali-Neutralisation der Haut wird positiv beeinflußt. Gleichwohl wirkt das Hautwaschmittel auch desodorierend.

Im übrigen erfolgt auch bei längerer Lagerung nur eine leichte Abnahme der waschaktiven Substanz.

Das mit dem erfindungsgemäßen Hautwaschmittel erzielte Ergebnis ist überraschend. Es wird dem dringenden Bedürfnis nach einem Hautwaschmittel Rechnung getragen, das hautschonend insbesondere im Hinblick auf die Vermeidung von Allergien ist.

In dieser Offenbarung verstehen sich alle Teil-Angaben und Prozentangaben zu den einzelnen Bestandteilen des Stoffgemisches bzw. des Hautwaschmittels bezogen auf das Gewicht derselben. Ein Teil eines Bestandteiles bedeutet also ein Gewichtsteil, ein Prozent eines Bestandteiles ein Gewichtsprozent.

## Patentansprüche

1. Stoffgemisch enthaltend Alkylsulfosuccinat, Fettalkoholpolyglykoläthersuccinat, Fettsäurealkylolamid, Konservierungsmittel und Wasser, dadurch gekennzeichnet, daß es aus

a) 450 bis 600 Teilen Dinatriumfettalkoholpolyglykoläthersulfosuccinat der Struktur,

$$R-(-O-CH_2-CH_2-)_n-O-CO-CH_2-\underset{SO_3Na}{CH}-COONa$$

mit n insbesondere von 2 bis 12,

b) 30 bis 50 Teilen Dinatriumalkylsulfosuccinat, wobei sich der Alkylrest von einer pflanzlichen Fettsäure ableitet,

c) 30 bis 50 Teile nichtionogenes Fettsäurealkylolamid, insbesondere Cocosfettsäureisopropanolamid der Struktur

$$R-\overset{O}{\overset{\|}{C}}-NH-CH_2-\overset{OH}{CH}-CH_3$$

d) 20 bis 40 Teile Lanolin-Sulfosuccinat,

e) 1 Teil Zitronensäure,

f) 1 Teil Konservierungsmittel, frei von Formaldehyd und Formaldehydabspaltern und

g) zu 1 000 Teilen Wasser ergänzt besteht.

2. Verwendung des Stoffgemisches nach Anspruch 1 als Hautwaschmittel.

## Claims

1. A mixture of substances containing alkyl sulphosuccinate, fatty alcohol polyglycol ether succinate, fatty acid alkylolamide, preserving agent and water, characterised in that it comprises :

a) from 450 to 600 parts of disodium fatty alcohol polyglycol ether sulphosuccinate of the structure :

$$R-(-O-CH_2-CH_2-)_n-O-CO-CH_2-\underset{SO_3Na}{CH}-COONa$$

wherein n is in particular from 2 to 12,

b) from 30 to 50 parts of disodium alkyl sulphosuccinate, the alkyl residue being derived from a vegetable fatty acid,

c) from 30 to 50 parts of non-ionogenic fatty acid alkylolamide, in particular coconut fatty acid isopropanolamide having the structure :

$$R-\overset{O}{\overset{\|}{C}}-NH-CH_2-\overset{OH}{\overset{|}{C}H}-CH_3$$

d) from 20 to 40 parts of lanolin sulphosuccinate,

e) 1 part of citric acid,

f) 1 part of preserving agent, free from formaldehyde and formaldehyde separators, and

g) water, to make up 1 000 parts.

2. Use of the mixture of substances according to claim 1 as a skin washing agent.

**Revendications**

1. Mélange de substances contenant un alkylsulfosuccinate, un (alcool gras) poly(éther de glycol) succinate, un alkylolamide d'acide gras, un agent de conservation et de l'eau, mélange caractérisé en ce qu'il consiste en :

a) 450 à 600 parties d'alcool gras, poly(éther de glycol) sulfosuccinate disodique de structure :

$$R-(-O-CH_2-CH_2-)_n-O-CO-CH_2-\underset{SO_3Na}{\underset{|}{C}H}-COONa$$

dans laquelle n vaut en particulier 2 à 12,

b) 30 à 50 parties d'un alkylsulfosuccinate disodique, dans lequel le reste alkyle dérive d'un acide gras végétal,

c) 30 à 50 parties d'un alkylolamide d'acide gras non ionogène, en particulier de l'isopropanolamide d'acide gras de noix de coco de structure

$$R-\overset{O}{\overset{\|}{C}}-NH-CH_2-\overset{OH}{\overset{|}{C}H}-CH_3$$

d) 20 à 40 parties de lanoline-sulfosuccinate,

e) 1 partie d'acide citrique,

f) 1 partie d'un agent de conservation, dépourvu de formaldéhyde et de produits cédant du formaldéhyde, et

g) complété à 1 000 parties par de l'eau.

2. Utilisation du mélange des substances selon la revendication 1, à titre de produit pour le nettoyage de la peau.